# EUROPÄISCHE PATENTSCHRIFT

(11) **EP 2 205 299 B1**
(45) Veröffentlichungstag und Bekanntmachung des Hinweises auf die Patenterteilung: **02.11.2011**
(21) Anmeldenummer: 08832176.5
(22) Anmeldetag: 19.09.2008
(51) Int. Cl.: A61M 5/34, B01L 3/02, G01N 1/00

(54) **SPRITZE MIT WECHSELBARER NADEL**
SYRINGE WITH EXCHANGEABLE NEEDLE
SERINGUE À AIGUILLE INTERCHANGEABLE

(30) Priorität: 19.09.2007 DE 202007013096 U
(43) Veröffentlichungstag der Anmeldung: 14.07.2010
(73) Patentinhaber: Hamilton Bonaduz AG, 7402 Bonaduz (CH)
(72) Erfinder: LECKEBUSCH, Klaus, CH-7425 Masein (CH)
(74) Vertreter: Trossin, Hans-Jürgen
(86) Internationale Anmeldenummer: PCT/EP2008/007911
(87) Internationale Veröffentlichungsnummer: WO 2009/036994

(56) Entgegenhaltungen:
- WO-A-2004/047895
- WO-A-2006/096901

## Beschreibung

Die vorliegende Erfindung betrifft eine Spritze, wie sie etwa zur Probenbehandlung in der Chromatographie und dergleichen verwendet wird. Eine derartige Spritze weist einen Spritzenkörper auf, welcher eine in Richtung einer Spritzenkörper-Längsachse verlaufende axiale Innenausnehmung aufweist, in der ein Kolben axial beweglich geführt ist. Dieser Kolben trägt zusammen mit einem Abschnitt einer die Innenausnehmung in radialer Richtung begrenzenden Wandung zur Begrenzung eines Fluidaufnahmevolumens bei. Die Spritzenkörper-Längsachse definiert dabei grundsätzlich die axiale Richtung. Eine hierzu orthogonale Richtung ist eine radiale Richtung im Sinne der vorliegenden Erfindung.

Weiterhin weist eine derartige Spritze eine Nadel auf, welche an einem Längsende des Spritzenkörpers mit diesem verbindbar oder verbunden ist.

Es sind aus der Medizin grundsätzlich Spritzen bekannt, bei welchen eine Nadel an ihrem spritzenkörpernäheren Längsende eine Kopplungsgeometrie aufweist, durch welche die Nadel mit einer Gegenkopplungsgeometrie des Spritzenkörpers verbindbar ist. Dabei weist bei den aus dem Stand der Technik bekannten medizinischen Spritzen die Gegenkopplungsgeometrie einen Einführabschnitt auf, welcher im zusammengebauten Zustand der Spritze in einer Kopplungsausnehmung der Kopplungsgeometrie aufgenommen ist.

Die aus der Medizin bekannten Nadel-Spritzenkörper-Kopplungen sind für Anwendungen im Laborbereich nicht ausreichend. Sie erfüllen nicht die notwendigen Anforderungen an Festigkeit und vor allem Dichtigkeit, so dass die Reinheit eines von der Spritze aufzunehmenden und abzugebenden Fluids nicht in ausreichendem Maße gewährleistet ist.

WO2006/096901 beschreibt eine Spritze mit austauschbaser Nadel.

Es ist daher die Aufgabe der vorliegenden Erfindung, die aus dem Stand der Technik bekannten Spritzen derart weiterzubilden, dass die oben genannten Nachteile überwunden sind.

Gemäß der vorliegenden Erfindung wird diese Aufgabe gelöst durch eine Spritze gemäß Anspruch 1. Bevorzugte Ausführungsformen sind in den abhängigen Anspruchen beschrieben.

Durch die vorgesehenen Sicherungsmittel kann die Nadel nicht mehr vom Spritzenkörper entfernt werden, so dass die Nadel sicher mit dem Spritzenkörper gekoppelt ist.

Ein Sichern der Nadel am und ein Lösen derselben vom Spritzenkörper sowie ein besonders bevorzugtes Abdichten der Nadel-Spritzenkörper-Kopplung wird dadurch erreicht, dass die Sicherungsmittel ein Axialverstellungsbauteil aufweisen, welches durch Betätigung in axialer Richtung verlagerbar ist, so dass das Axialverstellungsbauteil axialen Druck auf den in die Kopplungsausnehmung eingeführten Einführabschnitt ausüben kann.

Spanngeometrie, welche im montierten Zustand der Spritze in Eingriff ist mit einer der Nadel zugeordneten Spanngegengeometrie.

Die Spanngeometrie und die Spanngegengeometrie können dann besonders einfach und sicher in Dichtungseingriff miteinander gebracht werden, wenn eine Geometrie aus Spanngeometrie und Spanngegengeometrie einen ersten Verjüngungsabschnitt aufweist, welcher sich zum freien Längsende der ihn tragenden Geometrie hin verjüngt und welcher im montierten Zustand der Spritze zumindest abschnittsweise in eine Aufnahmeausnehmung der jeweils anderen Geometrie eingeführt ist, und dass die Aufnahmeausnehmung einen ersten Aufweitungsabschnitt aufweist, welcher sich zum freien Längsende der ihn tragenden Geometrie aufweitet, wobei sich vorzugsweise der Verjüngungsverlauf des ersten Verjüngungsabschnitts in axialer Richtung und der Aufweitungsverlauf des ersten Aufweitungsabschnitts in axialer Richtung im Wesentlichen derart entsprechen, dass im montierten Zustand der Spritze der Verjüngungsabschnitt zumindest längs einer Umfangslinie um die Spritzenkörper-Längsachse linienförmig, besonders bevorzugt längs einer Umfangsberührfläche flächig an dem Aufweitungsabschnitt anliegt.

Dabei kann konstruktiv vorgesehen sein, dass der erste Verjüngungsabschnitt einen Konusabschnitt aufweist und vorzugsweise die Aufnahmeausnehmung eine konische Ausnehmung aufweist. Besonders bevorzugt weisen der Konusabschnitt und die konische Ausnehmung einen gleichen Konusöffnungswinkel auf.

Die Spanngeometrie ist an einem gesondert vom Axialverstellungsbauteil vorgesehenen SpannbAauteil ausgebildet, welches durch das Axialverstellungsbauteil axial verlagerbar ist. In diesem Falle kann nämlich die Relativbewegung gezielt an unkritischen Stellen herbeigeführt und zwischen Spritzenbauteilen an kritischen Stellen reduziert werden, was die Gefahr einer Bauteilbeschädigung reduziert.

Eine besonders gute Abdichtung der Nadel gegenüber dem Spritzenkörper und der Umgebung bei gleichzeitig sichergestellter korrekter Positionierung der Nadel relativ zum Spritzenkörper kann gemäß einer ersten Weiterbildung der vorliegenden Erfindung dadurch sichergestellt werden, dass ein die Spanngeometrie aufweisendes Bauteil zur gemeinsamen Bewegung mit der Nadel ausgebildet ist. Gemäß einer alternativen zweiten Weiterbildung der vorliegenden Erfindung kann zumindest die korrekte Positionierung der Nadel relativ zum Spritzenkörper auch dadurch sichergestellt werden, dass das Axialverstellungsbauteil zur gemeinsamen Bewegung mit der Nadel ausgebildet ist.

Die Ausbildung eines Bauteils "zur gemeinsamen Bewegung" mit der Nadel kann entweder durch einstückige Ausbildung des Bauteils mit der Nadel oder durch eine starre Verbindung des Bauteils mit der Nadel erreicht werden.

Zur vorteilhaften, möglichst exakten Positionierung des Axialverstellungsbauteils kann vorgesehen sein, dass dieses an einem Führungsbauteil zur axialen Bewegung geführt ist.

Aus konstruktiver Sicht kann das Führungsbauteil eine Hülse umfassen, welche am Spritzenkörper, insbesondere an dessen nadelnäherem Längsende, vorgesehen ist.

Zur besonders sicheren Verbindung des Führungsbauteils mit dem Spritzenkörper kann daran gedacht sein, dass das Führungsbauteil, insbesondere die Hülse, durch Pressen oder/und Verkleben mit dem Spritzenkörper verbunden ist.

Eine besonders einfache, sichere und gleichzeitig genaue Positionierung des Axialverstellungsbauteils relativ zum Führungsbauteil kann dadurch erreicht werden, dass das Führungsbauteil, insbesondere die Hülse, ein Gewinde, vorzugsweise Innengewinde umfasst, und dass das Axialverstellungsbauteil eine Schraube umfasst, welche ein mit dem Gewinde in Eingriff stehendes oder bringbares Gegengewinde, vorzugsweise Außengewinde aufweist. Dabei hängt die Positioniergenauigkeit von der gewählten Steigung des Gewindes und Gegengewindes ab.

Vorzugsweise ist das Gewinde des Führungsbauteils ein Innengewinde, so dass das Axialverstellungsbauteil in dieses eingedreht werden kann. Dies verhindert eine übermäßige radiale Abmessung der Spritze.

Zum vorteilhaften, weil an den jeweiligen Einsatzfall jeweils anpassbaren modularen Aufbau der Spritze kann vorgesehen sein, dass die Kopplungsgeometrie an einem gesonderten Kopplungsbauteil vorgesehen ist.

Für eine definierte Positionierung des Kopplungsbauteils in axialer Richtung und damit für die Sicherstellung einer möglichst guten Kopplungs- und Dichtungswirkung kann daran gedacht sein, dass das Kopplungsbauteil einen Vorsprung, vorzugsweise Radialvorsprung zur Anlage an einer Gegenanlagefläche des Spritzenkörpers aufweist.

Die Dichtungswirkung, welche mit dem Kopplungsbauteil erzielt werden kann, kann noch dadurch erhöht werden, dass es aus einem elastischen Werkstoff hergestellt ist. In diesem Falle kann der Einführabschnitt, sofern er am Kopplungsbauteil vorgesehen ist, einen zylindrischen Abschnitt aufweisen, zur Erleichterung der Einführung in die Kopplungsausnehmung, ggf. mit einer Einführschräge, wobei der zylindrische Abschnitt des Einführabschnitts dann in die Kopplungsausnehmung einzuführen ist und durch Verspannung durch die oben genannte Spanngeometrie sich dichtend an eine zugeordnete Wandung der Kopplungsausnehmung anlegt.

Ebenso kann daran gedacht sein, den Einführabschnitt mit einer bauchigen Außengestalt auszubilden, so dass bereits eine radiale Vorspannung aufgrund elastischer Materialverformung beim Einführen des Einführabschnitts in die Kopplungsausnehmung erzielt werden kann. Jedoch sollten Spalte an der Kopplungsstelle des Kopplungsbauteils mit dem Spritzenkörper vermieden werden, da sich hier Materialien ansammeln können, welche bei mehreren aufeinander folgenden Füllungen der Spritze das im dortigen Fluidaufnahmevolumen aufgenommene Fluid verunreinigen können.

Ebenso kann daran gedacht sein, das Kopplungsbauteil aus einem starren Werkstoff herzustellen und das starre Kopplungsbauteil dichtend in Anlage an eine ebenfalls starre Gegenkopplungsgeometrie am Spritzenkörper zu bringen.

Sowohl für den Fall, dass der Einführabschnitt und die Kopplungsausnehmung aus starren Materialien hergestellt sind, als auch für den Fall, dass wenigstens eine der Ausbildungen aus einem elastischnachgiebigen Werkstoff, wie etwa Polytetrafluorethylen, hergestellt ist, kann eine gute Dichtungswirkung der Kopplungsstelle zwischen Kopplungsgeometrie und Gegenkopplungsgeometrie dadurch erreicht werden, dass der Einführabschnitt einen zweiten Verjüngungsabschnitt aufweist, welcher sich zum freien Längsende der ihn tragenden Geometrie hin verjüngt und welcher im montierten Zustand der Spritze zumindest abschnittsweise in die Kopplungsausnehmung der jeweils anderen Geometrie eingeführt ist, und dass die Kopplungsausnehmung einen zweiten Aufweitungsabschnitt aufweist, welcher sich zum freien Längsende der ihn tragenden Geometrie hin aufweitet, wobei sich vorzugsweise der Verjüngungsverlauf des zweiten Verjüngungsabschnitts in axialer Richtung und der Aufweitungsverlauf des zweiten Aufweitungsabschnitts in axialer Richtung im Wesentlichen derart entsprechen, dass im montierten Zustand der Spritze der zweite Verjüngungsabschnitt zumindest längs einer Umfangslinie um die Spritzenkörper-Längsachse linienförmig, besonders bevorzugt längs einer Umfangsberührfläche flächig an dem zweiten Aufweitungsabschnitt anliegt. Dabei können bereits geringe Verjüngungs- bzw. Aufweitungswinkel zu den gewünschten Kopplungs- und Dichtungsresultaten führen.

Aus fertigungstechnischer Sicht besonders einfach und daher bevorzugt weist der Einführabschnitt einen Konusabschnitt auf und weist vorzugsweise die Kopplungsausnehmung eine konische Ausnehmung auf.

Die Dichtungswirkung der Kopplungsstelle kann noch dadurch weiter erhöht werden, dass der Einführabschnitt eine Endfläche mit radialer Erstreckungskomponente aufweist, welche im montierten Zustand der Spritze an einer Gegenfläche des Spritzenkörpers dichtend anliegt. Dies empfiehlt sich jedoch zur Vermeidung von unerwünschten Doppelpassungen vor allem dann, wenn wenigstens eine der an der Kopplung beteiligten Geometrien aus elastischem Material hergestellt ist.

Gerade beim Einsatz der hier beschriebenen Spritze in Labors, insbesondere in der Chromatographie, ist die Vermeidung einer Verunreinigung des in der Spritze aufgezogenen Fluids von großer Wichtigkeit. Hierzu kann vorgesehen sein, dass wenigstens ein Teil der Flächen, vorzugsweise alle Flächen der Spritze, welche im Gebrauch derselben in Kontakt mit einem von der Spritze aufzunehmenden oder/und abzugebenden Fluid gelangen, mit einer Beschichtung versehen sind. Diese Beschichtung kann eine Quarz- oder Glasbeschichtung sein, welche eine besonders glätte, gegenüber zahlreichen Fluiden inerte und gleichzeitig transparente Oberfläche bereitstellen. Die Beschichtung kann durch die sogenannte Nano-Technologie auf der Innen- oder/und der Außenseite der Nadel aufgebracht sein. Die Beschichtung kann in an sich bekannter Weise durch einen Sol-Gel-Prozess an den in Frage kommenden Wänden bzw. Wandabschnitten der Spritze aufgebracht werden.

Die vorliegende Erfindung wird im Folgenden anhand der beiliegenden Zeichnungen näher beschrieben werden. Es stellt dar:
- Fig. 1: eine Teilschnittansicht einer ersten Ausführungsform einer erfindungsgemäßen Spritze,
- Fig. 2: eine Detail-Längsschnittansicht der Nadel-Spritzenkörper-Kopplung der ersten Ausführungsform von Fig. 1,
- Fig. 3: eine zweite Ausführungsform einer Nadel-Spritzenkörper-Kopplung und
- Fig. 4: eine dritte Ausführungsform einer Nadel-Spritzenkörper-Kopplung.

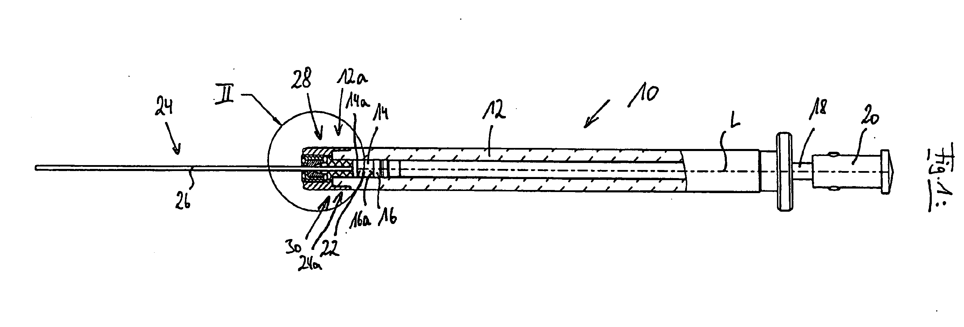

In Fig. 1 ist eine erste Ausführungsform einer erfindungsgemäßen Spritze allgemein mit 10 bezeichnet. Die Spritze 10 weist einen Spritzenkörper 12 auf mit einer in Richtung einer Spritzenkörper-Längsachse L verlaufenden Innenausnehmung 14, in welcher ein Kolben 16 in Richtung der Spritzenkörper-Längsachse L, also in axialer Richtung, beweglich aufgenommen ist.

Der Kolben 16 ist bewegbar durch eine starr mit dem Kolben 16 verbundene Kolbenstange 18, welche über ein Betätigungsendglied 20 zug- und druckbetätigt werden kann.

Eine zum nadelnäheren Längsende 12a des Spritzenkörpers 12 weisende Kolbenfläche 16a trägt zusammen mit einem Abschnitt 14a einer die Innenausnehmung 14 in radialer Richtung begrenzenden Umfangswandung zur Begrenzung eines Fluidaufnahmevolumens 22 bei.

Die in Fig. 1 gezeigte Spritze 10 umfasst weiterhin eine Nadel 24, mit einer Nadelkanüle 26. Die Nadel 24 weist an ihrem im montierten Zustand spritzenkörpernäheren Längsende 24a eine Kopplungsgeometrie 28 auf, welche mit einer Gegenkopplungsgeometrie 30 am nadelnäheren. Längsende 12a des Spritzenkörpers 12 zur Kopplung der Nadel 24 mit dem Spritzenkörper 12 in Eingriff ist.

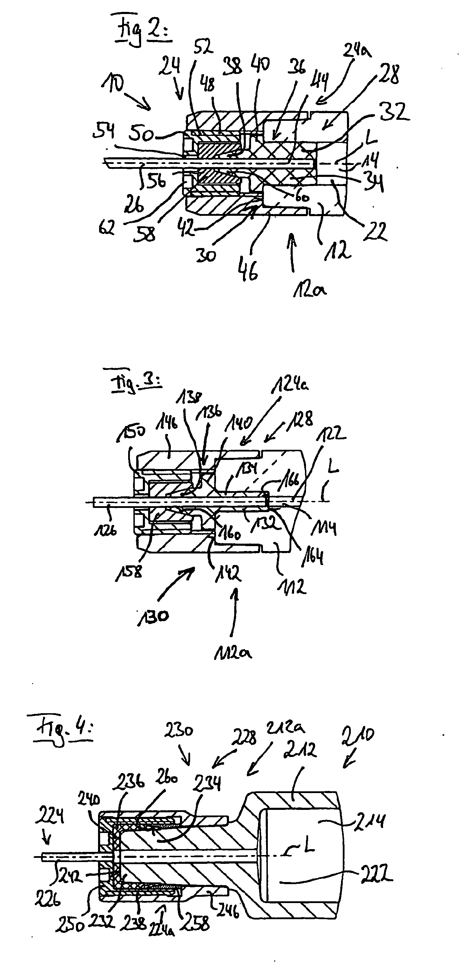

Die detaillierte Ausgestaltung der Kopplung der Nadel 24 mit dem Spritzenkörper 12 ist in Fig. 2 dargestellt. Fig. 2 zeigt das in Fig. 1 umkreiste und mit "11" bezeichnete Detail vergrößert.

In dem in Fig. 2 gezeigten ersten Ausführungsbeispiel umfasst die Kopplungsgeometrie 28 einen im Wesentlichen zylindrischen Einführabschnitt 32, welcher in eine Kopplungsausnehmung 34 der Gegenkopplungsgeometrie 30 eingeführt ist. Zur Erleichterung der Einführung des Einführabschnitts 32 in die Kopplungsausnehmung 34 ist der Einführabschnitt 32 an seinem in die Kopplungsausnehmung 34 eingeführten Längsende mit einer Einführschräge bzw. einer umlaufenden Einführfase versehen.

Der Einführabschnitt 32 ist integraler Bestandteil eines aus einem elastischen Kunststoff, im vorliegenden Beispiel Polytetrafluorethylen, hergestellten Kopplungsbauteils 36.

Dieses Bauteil 36 weist an seinem dem Einführabschnitt 32 entgegengesetzten Längsende eine Spanngegengeometrie 38 in Form eines Spannkonus auf.

Axial zwischen der Spanngegengeometrie 38 und dem Einführabschnitt 32 ist an dem Kopplungsbauteil 36 ein in Umfangsrichtung umlaufender Radialvorsprung 40 ausgebildet, welcher im montierten Zustand der Spritze 10 an einer Gegenanlagefläche 42 des Spritzenkörpers 12 anliegt. Wenn der Radialvorsprung 40 zur Anlage an die Gegenanlagefläche 42 kommt, ist ein korrekter Sitz des Kopplungsbauteils 36 sichergestellt.

In einer zentralen durchgehenden Ausnehmung 44 des Kopplungsbauteils 36 ist die Nadelkanüle 26 eingeschoben.

Grundsätzlich kann die Nadelkanüle 26 mit dem Kopplungsbauteil 36 durch Kleben oder dergleichen verbunden sein. Bevorzugt ist zur Vermeidung von unerwünschten Verunreinigungen eines im Fluidaufnahmeraum 22 aufgenommenen Fluids durch Klebemittelbestandteile die Nadelkanüle 26 lediglich kraft- bzw. reibschlüssig im Kopplungsbauteil 36 gehalten.

Auf einen Radialabsatz am Längsende 12a des Spritzenkörpers 12 ist ein hülsenförmiges Führungsbauteil 46 durch Aufpressen und Verkleben mit dem Spritzenkörper 12 verbunden.

Das Führungsbauteil 46 weist ein Innengewinde 48 auf, in welches ein Axialverstellungsbauteil in Form einer Spannschraube 50 mit einem Außengewinde 52 längs der Spritzenkörper-Längsachse L axial beweglich eingeschraubt ist.

Durch eine zentrale Ausnehmung 54 ist die Nadelkanüle 26 hindurchgeführt, wobei der Durchmesser der zentralen Ausnehmung 54 größer als der Außendurchmesser der Nadelkanüle 26 ist.

In einer topfförmigen Ausnehmung 56 der Spannschraube 50 ist ein Spannbauteil 58 aufgenommen, welches mit der Nadelkanüle 26 zur gemeinsamen Bewegung verklebt, verschweißt oder sonstwie verbunden ist.

Das Spannbauteil 58 weist eine Spanngeometrie in Form einer konischen Aufnahmeausnehmung 60 auf, in welche die konische Spanngegengeometrie 38 des Kopplungsbauteils 36 in axialer Richtung einragt.

Durch Drehen der Spannschraube 50 mittels eines in die Werkzeugeingriffsausnehmungen 62 an der spritzenkörperfernen Stirnseite der Spannschraube 50 zum Spritzenkörper 12 hin kann das elastische Kopplungsbauteil 36 gezielt elastisch verformt werden, so dass es spaltfrei dichtend am Kopplungsbauteil 12 anliegt.

In dem in Fig. 2 gezeigten ersten Ausführungsbeispiel geht die Kopplungsausnehmung 34 stufenlos in die Innenausnehmung 14 über.

In Fig. 3 ist ein alternatives zweites Ausführungsbeispiel dargestellt. Gleiche Bauteile wie in Fig. 2 sind mit gleichen Bezugszeichen versehen, jedoch erhöht um die Zahl 100.

Die Ausführungsform der Fig. 3 wird im Folgenden nur insoweit beschrieben werden, als sie sich von der Ausführungsform der Fig. 1 und 2 unterscheidet, auf deren Beschreibung ausdrücklich verwiesen wird.

Die zweite Ausführungsform, welche in Fig. 3 dargestellt ist, eignet sich für Spritzen mit äußerst geringen Spritzenvolumina, d.h. mit einer Innenausnehmung 114 mit sehr geringem Durchmesser.

Zu erkennen ist, dass die Kopplungsausnehmung 134 einen größeren Durchmesser aufweist als die Innenausnehmung 114 des Spritzenkörpers 112.

Daher geht die Kopplungsausnehmung 134 unter Bildung einer radialen Stufe in die Innenausnehmung 114 über. Somit kann eine stirnseitige Endfläche 164 des Einführabschnitts 132 an einer Gegenfläche 166 des Spritzenkörpers 112 dichtend zur Anlage kommen, wenn mittels der Spannschraube 150 und dem Spannbauteil 158 ausreichend Verformungsdruck auf das Kopplungsbauteil 136 ausgeübt wird.

In Fig. 4 ist eine dritte Ausführungsform einer erfindungsgemäßen Spritze mit einer Nadel-Spritzenkörper-Kopplung dargestellt.

Gleiche Bauteile wie in den Ausführungsformen der Fig. 1 bis 3 sind in der dritten Ausführungsform gemäß Fig. 4 mit den gleichen Bezugszeichen versehen, jedoch erhöht um die Zahl 200 bzw. 100. Die dritte Ausführungsform wird nur insofern beschrieben werden, als sie sich von den ersten beiden Ausführungsformen unterscheidet, auf deren Beschreibung ansonsten ausdrücklich verwiesen ist.

Bei der in Fig. 4 gezeigten Ausführungsform ist der Einführabschnitt 232 an der Gegenkopplungsgeometrie 230 des Spritzenkörpers 212 vorgesehen, während die Kopplungsausnehmung 234 an der Kopplungsgeometrie 228 der Nadel 224 ausgebildet ist.

Der Radialvorsprung 240 des Kopplungsbauteils 236 erstreckt sich im Gegensatz zu den vorherigen Ausführungsformen am spritzenkörperferneren Längsende des Kopplungsbauteils 236 nach radial innen.

Ebenfalls im Gegensatz zu den vorhergehenden Ausführungsformen ist das Spannbauteil 258 an dem hülsenförmigen Führungsbauteil 264 axial abgestützt.

Die Spannschraube 250 ist zur gemeinsamen Drehung mit der Nadelkanüle 226 verbunden.

Die Spanngegengeometrie 238 des Kopplungsbauteils 236 umgibt den Einführabschnitt 234 des Spritzenkörpers 212 radial außen in Umfangsrichtung und liegt an der konischen Aufnahmeausnehmung 260 des Spannbauteils 258 an.

## Patentansprüche

1. Spritze mit einem Spritzenkörper (12; 112; 212), welcher eine in Richtung einer Spritzenkörper-Längsachse (L) verlaufende axiale Innenausnehmung (14; 114; 214) aufweist, in der ein Kolben (16) axial beweglich geführt ist, welcher zusammen mit einem Abschnitt einer die Innenausnehmung (14) radial begrenzenden Wandung (14a) zur Begrenzung eines Fluidaufnahmevolumens beiträgt, und mit einer Nadel (24), welche an einem Längsende (12a; 112a; 212a) des Spritzenkörpers (12; 112; 212) mit diesem verbindbar oder verbunden ist,
wobei die Nadel (24; 124; 224) an ihrem spritzenkörpernäheren Längsende (24a; 124a; 224a) eine Kopplungsgeometrie (28; 128; 228) aufweist, durch welche die Nadel (24; 124; 224) mit einer Gegenkopplungsgeometrie (30; 130; 230) des Spritzenkörpers (12; 112; 212) verbindbar ist, wobei eine Geometrie aus Kopplungsgeometrie (28; 128; 228) und Gegenkopplungsgeometrie (30; 130; 230) einen Einführabschnitt (32; 132; 232) aufweist, welcher im zusammengebauten Zustand der Spritze (10; 110; 210) in einer Kopplungsausnehmung (34; 134; 234) der jeweils anderen Geometrie aufgenommen ist und wobei ferner Sicherungsmittel (46, 50; 146, 150; 246, 250) vorgesehen sind, welche im zusammengebauten Zustand der Spritze (10; 110; 210) eine Ausziehbewegung des Einführabschnitts (32; 132; 232) aus der Kopplungsausnehmung (34; 134; 234) verhindern,
wobei die Sicherungsmittel (46, 50; 146, 150; 246, 250) ein Axialverstellungsbautell (50; 150; 250) aufweisen, welches durch Betätigung in axialer Richtung verlagerbar ist,
wobei die Sicherungsmittel (46, 50; 146, 150; 246, 250) eine Spanngeometrie (60; 160; 260) umfassen, welche im montierten Zustand der Spritze (10; 110; 210) in Eingriff ist mit einer der Nadel (24) zugeordneten Spanngegengeometrie (38; 138; 238),
wobei die Spanngeometrie (60; 160; 260) an einem gesondert vom Axialverstellungsbauteil (50; 150; 250) vorgesehenen Spannbauteil (58; 158; 258) ausgebildet ist,
**dadurch gekennzeichnet, dass**
das Spannbauteil (58; 158; 258) durch das Axialverstellungsbauteil (50; 150; 250) axial verlagerbar ist.

2. Spritze nach Anspruch 1,
**dadurch gekennzeichnet, dass** eine Geometrie aus Spanngeometrie (60; 160; 260) und Spanngegengeometrie (38; 138; 238) einen ersten Verjüngungsabschnitt (38; 138; 238) aufweist, welcher sich zum freien Längsende der ihn tragenden Geometrie hin verjüngt und welcher im montierten Zustand der Spritze (10; 110; 210) zumindest abschnittsweise in eine Aufnahmeausnehmung (60; 160; 260) der jeweils anderen Geometrie eingeführt ist, und dass die Aufnahmeausnehmung (60; 160; 260) einen ersten Aufweitungsabschnitt aufweist, welcher sich zum freien Längsende der ihn tragenden Geometrie aufweitet, wobei sich vorzugsweise der Verjüngungsverlauf des ersten Verjüngungsabschnitts (38; 138; 238) in axialer Richtung und, der Aufweitungsverlauf des ersten Aufweitungsabschnitts in axialer Richtung im Wesentlichen derart entsprechen, dass im montierten Zustand der Spritze (10; 110; 210) der Verjüngungsabschnitt (38; 138; 238) zumindest längs einer Umfangslinie um die Spritzenkörper-Längsachse (L) linienförmig, besonders bevorzugt längs einer Umfangsberührfläche flächig an dem Autweitungsabschnitt anliegt.

3. Spritze nach Anspruch 2,
**dadurch gekennzeichnet, dass** der erste Verjüngungsabschnitt (38; 138; 238) einen Konusabschnitt aufweist und vorzugsweise die Aufnahmeausnehmung eine konische Ausnehmung (60; 160; 260) aufweist.

4. Spritze nach einem der vorhergehenden Ansprüche,
**dadurch gekennzeichnet, dass** ein die Spanngeometrie (60; 160; 260) aufweisendes Bauteil (58; 158) zur gemeinsamen Bewegung mit der Nadel (224) ausgebildet ist.

5. Spritze nach einem der vorhergehenden Ansprüche,
**dadurch gekennzeichnet, dass** das Axialverstellungsbauteil (50; 150; 250) zur gemeinsamen Bewegung mit der Nadel (224) ausgebildet ist.

6. Spritze nach einem der vorhergehenden Ansprüche,
**dadurch gekennzeichnet, dass** das Axialverstellungsbauteil (50; 150; 250) an einem Führungsbauteil (46; 146; 246) zur axialen Bewegung geführt ist.

7. Spritze nach Anspruch 6,
**dadurch gekennzeichnet, dass** das Führungsbauteil (46; 146; 246) eine Hülse umfasst, welche am Spritzenkörper (12; 112; 212), insbesondere an dessen nadelnäherem Längsende (12a; 112a; 212a), vorgesehen ist.

8. Spritze nach Anspruch 6 oder 7,
**dadurch gekennzeichnet, dass** das Führungsbauteil (46; 146; 246), insbesondere die Hülse, durch Pressen oder/und Verkleben mit dem Spritzenkörper (12; 112; 212) verbunden ist.

9. Spritze nach einem der Ansprüche 6 bis 8,
**dadurch gekennzeichnet, dass** das Führungsbauteil (46; 146; 246), insbesondere die Hülse, ein Gewinde (48; 148; 248), vorzugsweise Innengewinde (48; 148; 248) umfasst, und dass das Axialverstellungsbauteil (50; 150; 250) eine Schraube (50; 150; 250) umfasst, welche ein mit dem Gewinde (48; 148; 248) in Eingriff stehendes oder bringbares Gegengewinde (52; 152; 252), vorzugsweise Außengewinde (52; 152; 252) aufweist.

10. Spritze nach einem der vorhergehenden Ansprüche,
**dadurch gekennzeichnet, dass** die Kopplungsgeometrie (28; 128; 228) an einem gesonderten Kopplungsbauteil (36; 136; 236) vorgesehen ist.

11. Spritze nach Anspruch 10,
**dadurch gekennzeichnet, dass** das Kopplungsbauteil (36; 136; 236) einen Vorsprung (40; 140; 240), vorzugsweise Radialvorsprung (40; 140; 240) zur Anlage an einer Gegenanlagefläche (42; 142; 242) des Spritzenkörpers (12; 112; 212) aufweist.

12. Spritze nach einem der Ansprüche 10 oder 11,
**dadurch gekennzeichnet, dass** das Kopplungsbauteil (36; 136; 236) aus einem elastischen Werkstoff hergestellt ist.

13. Spritze nach einem der vorhergehenden Ansprüche,
**dadurch gekennzeichnet, dass** der Einführabschnitt (32; 132; 232) einen zweiten Verjüngungsabschnitt aufweist, welcher sich zum freien Längsende der ihn tragenden Geometrie hin verjüngt und weicher im montierten Zustand der Spritze (10; 110; 210) zumindest abschnittsweise in die Kopplungsausnehmung (34; 134; 234) der jeweils anderen Geometrie eingeführt ist, und dass die Kopplungsausnehmung (34; 134; 234) einen zweiten Aufinreitungsabschnitt aufweist, welcher sich zum freien Längsende der ihn tragenden Geometrie hin aufweitet, wobei sich vorzugsweise der Verjüngungsverlauf des zweiten Verjüngungsabschnitts in axialer Richtung und der Aufweitungsverlauf des zweiten Aufweitungsabschnitts in axialer Richtung im Wesentlichen derart entsprechen, dass im montierten Zustand der Spritze (10; 110; 210) der zweite Verjüngungsabschnitt zumindest längs einer Umfangslinie um die Spritzenkörper-Längsachse (L) linienförmig, besonders bevorzugt längs einer Umfangsberührfläche flächig an dem zweiten Aufweitungsabschnitt Anliegt.

14. Spritze nach Anspruch 13,
**dadurch gekennzeichnet, dass** der Einführabschnitt (32; 132; 232) einen Konusabschnitt aufweist und vorzugsweise die Kopplungsausnehmung (34; 134; 234) eine konische Ausnehmung aufweist.

15. Spritze nach einem der vorhergehenden Ansprüche,
**dadurch gekennzeichnet, dass** der Einführabschnitt (132) eine Endfläche (164) mit radialer Erstreckungskomponente aufweist, welche im montierten Zustand der Spritze (110) an einer Gegenfläche (166) des Spritzenkörpers (112) dichtend anliegt.

## Claims

1. Syringe comprising a syringe body (12; 112; 212), which comprises an axial internal recess (14; 114; 214) extending in the direction of a longitudinal axis (L) of the syringe body, in which recess a plunger (16) is guided in an axially movable manner, which plunger, together with a section of a wall (14a) radially defining the internal recess (14), contributes to the delimitation of a fluid take up volume, and comprising a needle (24), which at a longitudinal end (12a; 112a; 212a) of the syringe body (12; 112; 212), can be or is connected to the latter, wherein the needle (24; 124; 224) at its longitudinal end (24a; 124a; 224a) closer to its syringe body has a coupling geometry (28; 128; 228), by means of which the needle (24; 124; 224) can be connected to a mating coupling geometry (30; 130; 230) of the syringe body (12; 112; 212), wherein a geometry consisting of a coupling geometry (28; 128; 228) and mating connecting geometry (30; 130; 230) has an insertion section (32; 132; 232) which in the assembled state of the syringe (10; 110; 210) is accommodated in a coupling recess (34; 134; 234) of the respective other geometry, and wherein furthermore securing means (46, 50; 146, 150; 246, 250) are also provided, which prevent a withdrawal movement of the insertion section (32; 132; 232) out of the coupling recess (34; 134; 234) in the assembled state of the syringe (10; 110; 210), wherein the securing means (46, 50; 146, 150; 246, 250) comprise an axial adjustment component (50; 150; 250), which can be displaced by activation in axial direction, wherein the securing means (46, 50; 146, 150; 246, 250) have a tensioning geometry (60; 160; 260) which in the assembled state of the syringe (10; 110; 210) is in engagement with a mating tensioning geometry (38; 138; 238) assigned to the needle (24), wherein the tensioning geometry (60; 160; 260) is formed on a tensioning component (58; 158; 258) provided separately from the axial adjustment component (50; 150; 250),
**characterised in that** the tensioning component (58; 158; 258) can be displaced axially by the axial adjustment component (50; 150; 250).

2. Syringe according to claim 1,
**characterised in that** a geometry consisting of tensioning geometry (60; 160; 260) and mating tensioning geometry (38; 138; 238) comprises a first tapered section (38; 138; 238), which tapers towards the free longitudinal end of the geometry supporting it and which in the assembled state of the syringe (10; 110; 210) is introduced at least in sections into an accommodating recess (60; 160; 260) of the respective other geometry, and **in that** the accommodating recess (60; 160; 260) comprises a first widened section, which widens towards the free longitudinal end of the geometry supporting it, wherein preferably the tapering profile of the first tapered section (38; 138; 238) in axial direction and the widening profile of the first widened section in axial direction correspond to one another substantially such that in the assembled state of the syringe (10; 110; 210) the tapered section (38; 138; 238) bears in linear manner at least along a circumferential line around the syringe body longitudinal axis (L), particularly preferably bears along a circumferential contact surface flat against the widened section.

3. Syringe according to claim 2,
**characterised in that** the first tapered section (38; 138; 238) has a conical section and preferably the accommodating recess has a conical recess (60; 160; 260).

4. Syringe according to one of the preceding claims,
**characterised in that** a component (58; 158) with the tensioning geometry (60; 160; 260) is configured for joint movement with the needle (224).

5. Syringe according to one of the preceding claims,
**characterised in that** the axial adjustment component (50; 150; 250) is configured for joint movement with the needle (224).

6. Syringe according to one of the preceding claims,
**characterised in that** the axial adjustment component (50; 150; 250) is guided on a guiding component (46; 146; 246) for axial movement.

7. Syringe according to claim 6,
**characterised in that** the guiding component (46; 146; 246) comprises a sleeve, which is provided on the syringe body (12; 112; 212), in particular on its longitudinal end (12a; 112a; 212a) closer to the needle.

8. Syringe according to claim 6 or 7,
**characterised in that** the guiding component (46; 146; 246), in particular the sleeve, is connected to the syringe body (12; 112; 212) by pressing and/or adhesive bonding.

9. Syringe according to one of claims 6 to 8,
**characterised in that** the guiding component (46; 146; 246), in particular the sleeve, comprises a thread (48; 148; 248), preferably an internal thread (48; 148; 248), and **in that** the axial adjustment component (50; 150; 250) comprises a screw (50; 150; 250), which comprises a mating thread (52; 152; 252), preferably an external thread (52; 152; 252), which is in engagement with or capable of being brought into engagement with said thread (48; 148; 248).

10. Syringe according to one of the preceding claims,
**characterised in that** the coupling geometry (28; 128; 228) is provided on a separate coupling component (36; 136; 236).

11. Syringe according to claim 10,
**characterised in that** the coupling component (36; 136; 236) comprises a projection (40; 140; 240), preferably a radial projection (40; 140; 240), for resting against a mating contact face (42; 142; 242) of the syringe body (12; 112; 212).

12. Syringe according to one of claims 10 or 11,
**characterised in that** the coupling component (36; 136; 236) is made from a resilient material.

13. Syringe according to one of the preceding claims,
**characterised in that** the insertion section (32; 132; 232) comprises a second tapered section, which tapers towards the free longitudinal end of the geometry supporting it and which is inserted at least in sections into the coupling recess (34; 134; 234) of the respective other geometry when the syringe (10; 110; 210) is in the assembled state, and **in that** the coupling recess (34; 134; 234) comprises a second widened section, which widens towards the free longitudinal end of the geometry supporting it, wherein preferably the tapering profile of the second tapered section in axial direction and the widening profile of the second widened section in axial direction correspond to one another substantially such that in the assembled state of the syringe (10; 110; 210) the second tapered section bears in linear manner at least along a circumferential line around the syringe body longitudinal axis (L), particularly preferably bears flat along a circumferential contact surface against the second widened section.

14. Syringe according to claim 13,
**characterised in that** the insertion section (32; 132; 232) has a conical section and preferably the coupling recess (34; 134; 234) has a conical recess.

15. Syringe according to one of the preceding claims,
**characterised in that** the insertion section (132) comprises an end face (164) with a radial extension component, which bears in a sealing manner against a mating face (166) of the syringe body (112) when the syringe (110) is in the assembled state.

## Revendications

1. Seringue comprenant un corps de seringue (12 ; 112 ; 212), lequel présente une cavité intérieure (14 ; 114 ; 214) axiale, agencée en direction d'un axe longitudinal du corps de seringue (L), cavité dans laquelle un piston (16) est guidé de façon mobile axialement, lequel contribue en coopération avec un tronçon d'une paroi (14a) délimitant radialement la cavité intérieure (14) à la délimitation d'un volume de logement de fluide, et une aiguille (24), qui peut être reliée ou est reliée par une extrémité longitudinale (12a ; 112a ; 212a) du corps de seringue (12 ; 112 ; 212) à celui-ci, l'aiguille (24 ; 124 ; 224) présentant sur son extrémité longitudinale (24a ; 124a ; 224a) plus proche du corps de seringue une géométrie d'accouplement (28 ; 128 ; 228), par laquelle l'aiguille (24 ; 124 ; 224) peut être reliée à une géométrie de contre-accouplement (30 ; 130 ; 230) du corps de seringue (12 ; 112 ; 212), une géométrie constituée de la géométrie d'accouplement (28 ; 128 ; 228) et de la géométrie de contre-accouplement (30 ; 130 ; 230) présentant un tronçon d'introduction (32 ; 132 ; 232), qui est réceptionné, lorsque la seringue (10 ; 110 ; 210) est montée, dans une cavité d'accouplement (34 ; 134 ; 234) de l'autre géométrie respective et des moyens de blocage (46, 50 ; 146, 150 ; 246, 250) étant également prévus, lesquels empêchent, lorsque la seringue (10 ; 110 ; 210) est montée, un mouvement d'extraction du tronçon d'introduction (32 ; 132 ; 232) de la cavité d'accouplement (34 ; 134 ; 234), les moyens de blocage (46, 50 ; 146, 150 ; 246, 250) présentant un composant de réglage axial (50 ; 150 ; 250), qui peut être déplacé par actionnement dans le sens axial, les moyens de blocage (46, 50 ; 146, 150 ; 246, 250) comprenant une géométrie de serrage (60 ; 160 ; 260), qui est en prise, lorsque la seringue (10 ; 110 ; 210) est montée, avec une contre-géométrie de serrage (38 ; 138 ; 238) attribuée à l'aiguille (24), la géométrie de serrage (60 ; 160 ; 260) étant conçue sur un composant de serrage (58 ; 158 ; 258) prévu séparément du composant de réglage axial (50 ; 150 ;250),
**caractérisée en ce que**
le composant de serrage (58 ; 158 ; 258) peut être déplacé axialement par le composant de réglage axial (50 ; 150 ; 250).

2. Seringue selon la revendication 1,
**caractérisée en ce qu'**une géométrie constituée de la géométrie de serrage (60 ; 160 ; 260) et de la contre-géométrie de serrage (38 ; 138 ; 238) présente un premier tronçon de rétrécissement (38 ; 138 ; 238), qui se rétrécit en direction de l'extrémité longitudinale libre de la géométrie qui le porte et qui est introduit, lorsque la seringue (10 ; 110 ; 210) est montée, au moins par endroits dans une cavité de logement (60 ; 160 ; 260) de l'autre géométrie respective, et **en ce que** la cavité de logement (60 ; 160 ; 260) présente un premier tronçon d'élargissement, qui s'élargit en direction de l'extrémité libre longitudinale de la géométrie qui le porte, le tracé de rétrécissement du premier tronçon de rétrécissement (38 ; 138 ; 238) dans le sens axial et le tracé d'élargissement du premier tronçon d'élargissement dans le sens axial se correspondant sensiblement de préférence, de telle sorte que, lorsque la seringue (10 ; 110 ; 210) est montée, le tronçon de rétrécissement (38 ; 138 ; 238) s'applique au moins le long d'une ligne périphérique autour de l'axe longitudinal du corps de seringue (L) en forme de ligne, avec une préférence particulière, le long d'une surface de contact périphérique à plat sur le tronçon d'élargissement.

3. Seringue selon la revendication 2,
**caractérisée en ce que** le premier tronçon de rétrécissement (38 ; 138 ; 238) présente un tronçon de cône et la cavité de logement présente de préférence une cavité (60 ; 160 ; 260) conique.

4. Seringue selon l'une quelconque des revendications précédentes,
**caractérisée en ce qu'**un composant (58 ; 158) présentant la géométrie de serrage (60 ; 160 ; 260) est conçu pour le déplacement commun avec l'aiguille (224).

5. Seringue selon l'une quelconque des revendications précédentes,
**caractérisée en ce que** le composant de réglage axial (50 ; 150 ; 250) est conçu pour le déplacement commun avec l'aiguille (224).

6. Seringue selon l'une quelconque des revendications précédentes,
**caractérisée en ce que** le composant de réglage axial (50 ; 150 ; 250) est guidé sur un composant de guidage (46 ; 146 ; 246) pour le déplacement axial.

7. Seringue selon la revendication 6,
**caractérisée en ce que** le composant de guidage (46 ; 146 ; 246) comprend un manchon, qui est prévu sur le corps de seringue (12 ; 112 ; 212), en particulier sur son extrémité longitudinale (12a ; 112a ; 212a) plus proche de l'aiguille.

8. Seringue selon la revendication 6 ou 7,
**caractérisée en ce que** le composant de guidage (46 ; 146 ; 246), en particulier le manchon, est relié par compression et/ou collage au corps de seringue (12 ; 112 ; 212).

9. Seringue selon l'une quelconque des revendications 6 à 8,
**caractérisée en ce que** le composant de guidage (46 ; 146 ; 246), en particulier le manchon, comprend un filetage (48 ; 148 ; 248), de préférence un filetage intérieur (48 ; 148 ; 248), et **en ce que** le composant de réglage axial (50 ; 150 ; 250) comprend une vis (50 ; 150 ; 250), qui présente un contre-filetage (52 ; 152 ; 252), de préférence un filetage extérieur (52 ; 152 ; 252), qui est en prise ou peut être mis en prise avec le filetage (48 ; 148 ; 248).

10. Seringue selon l'une quelconque des revendications précédentes,
**caractérisée en ce que** la géométrie d'accouplement (28 ; 128 ; 228) est prévue sur un composant d'accouplement (36 ; 136 ; 236) séparé.

11. Seringue selon la revendication 10,
**caractérisée en ce que** le composant d'accouplement (36 ; 136 ; 236) présente une saillie (40 ; 140 ; 240), de préférence une saillie radiale (40 ; 140 ; 240), pour l'appui sur une surface de contre-appui (42 ; 142 ; 242) du corps de seringue (12 ; 112 ; 212).

12. Seringue selon l'une quelconque des revendications 10 ou 11,
**caractérisée en ce que** le composant d'accouplement (36 ; 136 ; 236) est fabriqué à base d'un matériau élastique.

13. Seringue selon l'une quelconque des revendications précédentes,
**caractérisée en ce que** le tronçon d'introduction (32 ; 132 ; 232) présente un second tronçon de rétrécissement, qui se rétrécit en direction de l'extrémité longitudinale libre de la géométrie qui le porte et qui, lorsque la seringue (10 ; 110 ; 210) est montée, est introduit au moins par endroits dans la cavité d'accouplement (34 ; 134 ; 234) de l'autre géométrie respective, et **en ce que** la cavité d'accouplement (34 ; 134 ; 234) présente un second tronçon d'élargissement, qui s'élargit en direction de l'extrémité longitudinale libre de la géométrie qui le porte, le tracé de rétrécissement du second tronçon de rétrécissement dans le sens axial et le tracé d'élargissement du second tronçon d'élargissement dans le sens radial se correspondant sensiblement de préférence, de telle sorte que, lorsque la seringue (10 ; 110 ; 210) est montée, le second tronçon de rétrécissement s'applique au moins le long d'une ligne périphérique autour de l'axe longitudinal du corps de seringue (L) en forme de ligne, avec une préférence particulière le long d'une surface de contact périphérique à plat sur le second tronçon d'élargissement.

14. Seringue selon la revendication 13,
**caractérisée en ce que** le tronçon d'introduction (32 ; 132 ; 232) présente un tronçon de cône et la cavité d'accouplement (34 ; 134 ; 234) présente de préférence une cavité conique.

15. Seringue selon l'une quelconque des revendications précédentes,
**caractérisée en ce que** le tronçon d'introduction (132) présente une surface d'extrémité (164) avec une composante d'extension radiale, laquelle s'applique de façon étanche sur une contre-surface (166) du corps de seringue (112), lorsque la seringue (110) est montée.
